# EUROPÄISCHE PATENTANMELDUNG

(11) **EP 4 054 202 A2**
(43) Veröffentlichungstag der Anmeldung: **07.09.2022**
(21) Anmeldenummer: 22157379.3
(22) Anmeldetag: 18.02.2022
(51) Int. Cl.: H04Q 9/00, G01N 33/00, G08B 21/14

(54) **SYSTEM ZUR AUSWERTUNG VON DATEN SOWIE MOBILES GASMESSGERÄT SOWIE DATENVERARBEITUNGSEINHEIT FÜR EIN DERARTIGES SYSTEM**

(30) Priorität: 02.03.2021 DE 102021105015
(71) Anmelder: Dräger Safety AG & Co. KGaA, 23560 Lübeck (DE)
(72) Erfinder: Berndt, Malte, 23558 Lübeck (DE); Rodehorst, Christof, 23558 Lübeck (DE); Maas, Raphael, 23558 Lübeck (DE)
(74) Vertreter: Guthöhrlein, Gerhard

(57) **Zusammenfassung**

Beschrieben wird ein System zur Auswertung von Daten sowie ein mobiles Gasmessgerät (2) um eine Datenverarbeitungseinheit (1) für ein derartiges System. Das System verfügt über wenigstens ein Gasmessgerät (2), das eingerichtet ist, um aktuelle Daten und/oder historische Daten, die in einem Datenspeicher (3) des Gasmessgeräts (2) abgelegt sind und eine Information über eine Gasmessgerätzeit zum Erfassungszeitpunkt der historischen Daten enthalten, bereitzustellen und drahtgebunden oder drahtlos an eine Schnittstelle (4) zu übertragen, die ausgeführt ist, um die aktuellen und/oder die historischen Daten zu empfangen und einer Datenverarbeitungseinheit (1) und/oder einem weiteren Datenspeicher (5) zuzuführen, und mit einer Datenkennzeichnungseinheit (6), die ausgeführt ist, um den von dem Gasmessgerät (2) bereitgestellten Daten wenigstens zeitweise eine spezifische Kennzeichnung hinzufügen,
dadurch gekennzeichnet, dass die Datenkennzeichnungseinheit (6) eingerichtet ist, um den von dem Gasmessgerät (2) bereitgestellten Daten eine Information über eine Systemzeit während der Datenübertragung und/oder die Gasmessgerätezeit während der Datenübertragung hinzuzufügen und dass die Datenverarbeitungseinheit (1) derart ausgeführt ist, dass eine Verarbeitung der vom Gasmessgerät (2) und/oder dem weiteren Datenspeicher (5) bereitgestellten Daten unter Berücksichtigung der Systemzeit und der Gasmessgerätezeit erfolgt.

## Beschreibung

Die Erfindung betrifft ein System zur Auswertung von Daten sowie ein mobiles Gasmessgerät und eine Datenverarbeitungseinheit für ein derartiges System. Das System verfügt über wenigstens ein Gasmessgerät, das eingerichtet ist, um aktuelle und/oder historische Daten, die in einem Datenspeicher des Gasmessgerätes abgelegt sind und eine Information über wenigstens eine Gasmessgerätezeit zum Erfassungszeitpunkt der historischen Daten enthalten, bereitzustellen und drahtgebunden oder drahtlos an eine Schnittstelle zu übertragen, die ausgeführt ist, um die aktuellen und/oder die historischen Daten zu empfangen und einer Datenverarbeitungseinheit und/oder einem weiteren Datenspeicher zuzuführen können. Ferner ist eine Datenkennzeichnungseinheit vorgesehen, die ausgeführt ist, um den von dem Gasmessgerät bereitgestellten Daten wenigstens zeitweise eine spezifische Kennzeichnung hinzuzufügen.

Aus dem Stand der Technik sind unterschiedliche Systeme, insbesondere Überwachungssysteme für Industrieanlagen, wie Raffinerien, Chemiewerke und Bergwerke, bekannt, die über mobile und stationäre Gasmessgeräte verfügen, welche Konzentrationen verschiedener Gase in einem Messbereich erfassen und so Umgebungsparameter detektieren und bei unzulässigen Abweichungen Alarmsignale generieren. Erkennt ein Gasmessgerät eine Grenzwertüberschreitung führt dieses Gerät entweder selbst eine Alarmierung durch oder sendet ein Alarmsignal an eine zentrale Datenverarbeitungseinheit, die oft Teil einer Leitwarte ist. Vielfach kommen mobile oder tragbare Gasmessgeräte zum persönlichen Schutz von Personen zum Einsatz, die den Geräteträger mithilfe eines Alarmsignals warnen, sofern toxische Gase oder Dämpfe eine gefährliche Konzentration in der unmittelbaren Umgebung des Geräteträgers annehmen, brennbare oder explosive Gasmischung vorhanden ist oder ein Mangel oder unzulässiger Überschuss an Sauerstoff besteht.

Die bekannten mobilen Gasmessgeräte verfügen üblicherweise über interne Datenspeicher, sogenannte Datenlogger, in denen sowohl erfasste Messwerte als auch bestimmte Ereignisse, wie etwa aufgetretene Fehler oder Alarme, abgespeichert werden. Diese auf dem Gerät gespeicherten Daten können genutzt werden, um bei Auftreten von Alarmen oder bei einem Störfall mit diesen Daten nachträgliche Analyse der Situation durchzuführen.

Unterdessen kommen immer mehr mobile Gasmessgeräte zum Einsatz, die die jeweils erzeugten Daten drahtlos an eine zentrale Datenverarbeitungseinheit oder einen zentralen Datenspeicher übertragen. Es ist somit möglich, die erfassten Messwerte und Informationen über im Messzeitraum erfolgte Alarme oder aufgetretene Fehler entweder in Echtzeit oder zumindest sobald eine Datenverbindung besteht zu übertragen und so vergleichsweise schnell für eine weitere Verarbeitung, beispielsweise in einer Leitwarte einer Industrieanlage, zur Verfügung zu stellen.

Werden derartige Gasmessgeräte, die drahtlos Messdaten oder sonstige Informationen übertragen, in bereits bestehenden Überwachungssystemen nachgerüstet, ergibts sich oftmals das Problem, dass unterschiedliche Arten von mobilen Gasmessgeräten, nämlich einerseits solche, die drahtlos Daten übertragen können und andererseits Geräte, die an eine Ausleseeinheit gekoppelt werden müssen, damit die auf diesen Geräten gespeicherten Daten übertragen werden können, nebeneinander genutzt werden. Werden in diesem Fall Daten von den unterschiedlichen Geräten an einen zentralen Datenspeicher oder eine zentrale Datenverarbeitungseinheit übertragen, stehen dem System sowohl historische als auch aktuelle Daten zur Verfügung. Es ist somit ein vergleichsweise großer Datenbestand von zu unterschiedlichen Zeitpunkten generierten Daten vorhanden, sodass bei einer Datenverarbeitung sichergestellt werden muss, dass die historischen und die aktuellen Daten in einen korrekten zeitlichen Zusammenhang zueinander gesetzt werden und eine mehrfache Berücksichtigung einzelner Datensätze vermieden wird.

Ausgehend von den aus dem Stand der Technik bekannten Lösungen zur Auswertung von Daten, die mithilfe von mobilen und/oder stationären Gasmessgeräten erzeugt und einer zentralen Datenverarbeitungseinheit zur Auswertung zugeführt werden, sowie den zuvor geschilderten Problemen liegt der Erfindung die Aufgabe zu Grunde, ein verbessertes System zur Auswertung von Daten bereitzustellen, insbesondere ein System zur Auswertung von Daten, die von mobilen und/oder stationären Gasmessgeräten erfasst und bereitgestellt werden, derart weiterzubilden, dass sowohl historische Daten als auch aktuelle, insbesondere in Echtzeit übertragene Daten, berücksichtigt werden und einer zuverlässigen Datenanalyse zur Verfügung stehen.

Durch Verwendung von zu unterschiedlichen Zeitpunkten generierten Daten, vor allem von historischen und aktuellen Daten, die von einem mobilen Gasmessgerät erfasst oder erstellt wurden, sollen Analysen durchführbar sein, die auf einem vergleichsweise großen Datenbestand beruhen und somit die Auswertung von Gefahrensituationen und Risikopotenzialen mit hoher Genauigkeit und Ortsauflösung ermöglichen. Hierfür ist es wünschenswert, wenn historische und aktuelle Daten miteinander korreliert werden können. Insbesondere soll somit die Genauigkeit von Analysen erhöht und eine größere örtliche Abdeckung bei der Überwachung eines Produktionsstandorts und somit die Bereitstellung eines vollständigeren Lagebildes realisiert werden. In diesem Zusammenhang ist es weiterhin wichtig, dass die erzeugten Daten möglichst zügig und in geordneter Form zur Verfügung stehen, um eine schnelle, genaue und zuverlässige Datenauswertung zu gewährleisten.

Von besonderer Bedeutung für genaue Analysen ist ferner, dass die mehrfache Berücksichtigung gleicher Datensätze vermieden und historische und aktuelle Daten bei ihrer Speicherung oder Verarbeitung in Bezug auf den Zeitpunkt ihrer Erfassung und Übertragung in eine korrekte zeitliche Reihenfolge gesetzt werden.

Im Weiteren sollte mit Hilfe der anzugebenden technischen Lösung eine effektive Nutzung der im Vergleich zu lokalen Datenspeichern großen Speicherkapazitäten von zentralen Datenspeichern sogenannter Cloud-Lösungen ermöglicht werden und ferner die Möglichkeit bestehen, den für eine Datenübertragung benötigten Zeitraum zu minimieren und/oder gezielt festzulegen oder aufzuteilen.

Die zuvor beschriebene Aufgabe wird mit einem System gemäß Anspruch 1, einem mobilen Gasmessgerät nach Anspruch 11 und einer Datenverarbeitungseinheit, die die im Anspruch 14 aufgeführten Merkmale aufweist, gelöst. Vorteilhafte Ausführungsformen der Erfindung sind Gegenstand der abhängigen Ansprüche und werden in der folgenden Beschreibung unter teilweiser Bezugnahme auf die Figuren näher erläutert.

Die Erfindung betrifft ein System zur Auswertung von Daten mit wenigstens einem Gasmessgerät, das eingerichtet ist, um aktuelle Daten und/oder historische Daten, die in einem Datenspeicher des Gasmessgeräts abgelegt sind und eine Information über eine Gasmessgerätzeit zum Erfassungszeitpunkt der historischen Daten enthalten, bereitzustellen und drahtgebunden oder drahtlos an eine Schnittstelle zu übertragen, die ausgeführt ist, um die aktuellen und/oder die historischen Daten zu empfangen und einer Datenverarbeitungseinheit und/oder einem weiteren Datenspeicher zuzuführen, und mit einer Datenkennzeichnungseinheit die ausgeführt ist, um den vom Gasmessgerät bereitgestellten Daten wenigstens zeitweise eine spezifische Kennzeichnung hinzuzufügen. Erfindungsgemäß ist das System derart weitergebildet worden, dass die Datenkennzeichnungseinheit eingerichtet ist, um den von dem Gasmessgerät bereitgestellten Daten eine Information über eine Systemzeit während der Datenübertragung und die Gasmessgerätezeit während der Datenübertragung hinzuzufügen und dass die Datenverarbeitungseinheit derart ausgeführt ist, dass eine Verarbeitung der vom Gasmessgerät und/oder dem weiteren Datenspeicher bereitgestellten Daten unter Berücksichtigung der Systemzeit und der Gasmessgerätezeit erfolgt.

Mithilfe des zuvor beschriebenen Systems zur Auswertung von Daten ist es somit möglich, sowohl historische als auch aktuelle Daten, die von einem Gasmessgerät, insbesondere einem mobilen oder tragbaren Gasmessgerät, bereitgestellt werden, zu nutzen. Vorzugsweise werden hierbei Informationen in Bezug auf wenigstens eine von einem Gasmessgerät erfasste Gaskonzentration, im Betrieb aufgetretene Fehler und/oder generierte Alarme übertragen und einer weiteren Datenverarbeitung und -analyse zur Verfügung gestellt. Die Daten werden hierbei in einer Form bereitgestellt, sodass eine Mehrfachnutzung gleicher Daten und/oder die Berücksichtigung falscher Erfassungszeitpunkte unterbleibt und zwar unabhängig davon, ob zumindest zeitweise nur aktuelle, nur historische oder gleichzeitig historische und aktuelle Daten übertragen werden. Durch das Vorsehen eines Zeitstempels der bevorzugt eine Information über die Gerätezeit zum Zeitpunkt der Datenerfassung und/oder -übertragung enthält, einerseits und einer Synchronisation der Erfassungszeitpunkte auf eine Systemzeit andererseits wird sichergestellt, dass sämtliche im System vorhandene Daten auf eine einheitliche Zeit synchronisiert sind. Damit ist gewährleistet, dass sowohl historische als auch aktuelle Daten in der Reihenfolge ihrer Entstehung berücksichtigt werden und/ oder korrekt den zu einem bestimmten Zeitpunkt stattgefundenen Ereignissen oder Fehlern zugeordnet werden.

Gemäß der Erfindung ist es weiterhin möglich, sowohl mobile oder tragbare Gasmessgeräte, die für eine drahtlose Datenübertragung eingerichtet sind, als auch solche, deren interner Datenspeicher lediglich zeitweise ausgelesen wird, in einem System gemeinsam mit stationären Gasmessgeräten, die wiederum Daten drahtlos und/oder drahtgebunden an eine zentrale Datenverarbeitungseinheit übertragen, zu nutzen. Das erfindungsgemäß ausgebildete System zur Auswertung von Daten ist derart eingerichtet, dass stets eine sichere Unterscheidung getroffen wird, welche Daten bereits an eine zentrale Datenverarbeitungseinheit und/oder einen zentralen Datenspeicher übertragen wurden, beispielsweise über eine kabellose Schnittstelle, und welche Daten noch übertragen werden müssen, etwa historische Daten, die bereits zu einem früheren Zeitpunkt generiert und auf dem internen Datenspeicher eines mobilen Gasmessgerätes abgespeichert sind und erst nach Verbinden mit einer speziellen Übertragungseinheit zur Datenverarbeitungseinheit übertragen werden.

Die Datenkennzeichnungseinheit, die eingerichtet ist, um den vom Gasmessgerät übertragenen Daten eine Information über die Gasmessgerätezeit sowie die Systemzeit zum Zeitpunkt der Datenübertragung hinzuzufügen, kann als separates Bauteil ausgeführt sein oder aber integraler Bestandteil einer anderen Komponente des erfindungsgemäßen Systems sein. So ist es generell denkbar, dass die Datenkennzeichnungseinheit Teil eines Gasmessgeräts, der zentralen Datenverarbeitungseinheit, einer Schnittstelle und/oder einer sonstigen für die Datenübertragung oder Datenverarbeitung vorgesehenen Systemkomponente ist.

Gemäß einer besonderen Ausführungsform der Erfindung ist die Schnittstelle und/oder die Datenkennzeichnungseinheit in ein Gateway integriert. Unter Gateway wird gemäß dieser Beschreibung ein Element zur Datenübertragung verstanden, über das eine Verbindung zwischen zwei Systemen oder einem System und einem Subsystem herstellbar ist. In diesem Zusammenhang ist es auf vorteilhafte Weise denkbar, dass durch das Gateway geleitete Daten im Gateway bearbeitet, insbesondere mit wenigstens einer weiteren Information angereichert werden. Gemäß einer speziellen Weiterbildung ist vorgesehen, dass die Schnittstelle eingerichtet ist, um beim Herstellen einer Datenverbindung zwischen dem Gasmessgerät und der Schnittstelle eine Information über die erfolgte Verbindungsherstellung an das Gasmessgerät zu übertragen. Vorzugsweise ist das Gasmessgerät in diesem Fall derart ausgebildet, dass die Information über die erfolgte Verbindungsherstellung im Datenspeicher des Gasmessgerätes abgespeichert wird. Im Weiteren ist es von Vorteil, dass bei Trennung der Datenverbindung zwischen dem Gasmessgerät und der Schnittstelle eine Information über die erfolgte Trennung im Gasmessgerät erzeugt und ebenfalls auf dem Datenspeicher des Gasmessgeräts gespeichert wird. Von Vorzug ist es, wenn einerseits die jeweilige Information über die erfolgte Verbindungsherstellung dem ersten daraufhin übertragenen Datensatz oder dem letzten nicht übertragenen Datensatz zugeordnet wird und andererseits die Trennung der Verbindung dem zuletzt übertragenen oder dem ersten in dem gerade abgeschlossenen Übertragungsintervall nicht mehr übertragenen Datensatz zugeordnet wird. Gemäß dieser speziellen Ausgestaltung ist im Datenspeicher eines Gasmessgeräts somit stets die Information vorhanden, ob eine Datenübertragungsverbindung zur zentralen Datenverarbeitungseinheit vorhanden ist und ob diese noch besteht oder bereits beendet wurde. Sobald die Datenübertragungsverbindung zwischen dem Gasmessgerät und der zentralen Datenverarbeitungseinheit wieder aktiv getrennt wird oder es zu einem Verbindungsabbruch kommt, wird dieser Zeitpunkt in Systemzeit oder in Gasmessgerätezeit vermerkt. So wird sichergestellt, dass die zwischen den beiden Einträgen der Zeitpunkte erzeugten Daten, die also erzeugt wurden, während eine Datenübertragungsverbindung bestand, nicht nochmals an die zentrale Datenverarbeitungseinheit übertragen oder zumindest von dieser nicht für eine weitere Bearbeitung und Auswertung berücksichtigt werden.

Gemäß einer speziellen Weiterbildung der Erfindung ist eine Kompensationseinheit vorgesehen, die eingerichtet, um den Erfassungszeitpunkt der historischen Daten und/oder deren Übertragungszeitpunkt und/oder den Übertragungszeitpunkt der aktuellen Daten, der in Gasmessgerätezeit vorliegt, unter Berücksichtigung der Systemzeit in den entsprechenden Zeitpunkt in Gasmessgerätezeit umzurechnen. Bevorzugt wird die Systemzeit zum Zeitpunkt der Erfassung und/oder Übertragung dem jeweils übertragenen Datensatz hinzugefügt. Bei einer derartigen technischen Lösung werden unterschiedliche Zeitzonen oder Zeitsysteme, die in einem derart vernetzten Überwachungssystem vorhanden sein können, berücksichtigt. Sobald die Gasmessgerätezeit nicht mit der Systemzeit übereinstimmt, werden die jeweils übertragenen Daten um die Information der Systemzeit zur Erfassungs- und/oder Übertragungszeitpunkt ergänzt oder der Datenbestandteil für die Gasmessgerätezeit mit der korrekten Systemzeit überschrieben. In diesem Zusammenhang ist es weiterhin denkbar, dass alle zur zentralen Datenverarbeitungseinheit übertragenen Daten mit einem Zeitstempel, der den Übertragungszeitpunkt in Systemzeit angibt, versehen werden. Um die Daten stets mit der Information über die korrekte Systemzeit anreichern zu können, wird die Kompensationseinheit bevorzugt mit der Systemzeit synchronisiert.

In einer besonderen Ausgestaltung der Erfindung werden für historische Daten, die bereits im System vorhanden sind und/oder von einem Gasmessgerät auf die zentrale Datenverarbeitungseinheit übertragen werden, die Erfassungszeitpunkte der Daten, die sich auf die Gasmessgerätzeit beziehen, kompensiert. Hierfür wird zum Zeitpunkt einer Datenübertragung sowohl die Gasmessgerätezeit aus einem Gasmessgerät ausgelesen als auch die aktuelle Systemzeit ermittelt und diese beiden Zeitinformationen dem ausgelesenen Datensatz hinzugefügt und gemeinsam mit diesem an die zentrale Datenverarbeitungseinheit übertragen. Auf der Grundlage der aktuellen Gasmessgerätezeit sowie der aktuellen Systemzeit ist es daraufhin möglich, nachträglich die Erfassungszeitpunkte der übertragenen Daten von der Gasmessgerätezeit in die Systemzeit umzurechnen.

Gemäß einer weiteren Ausgestaltung des erfindungsgemäßen Systems ist eine Komprimierungseinheit vorgesehen, die die von einem Gasmessgerät erfassten und/oder über die Schnittstelle zur Verfügung gestellten Daten in Abhängigkeit wenigstens eines Entscheidungskriteriums an die Datenverarbeitungseinheit und/oder den weiteren Datenspeicher weiterleitet. Auf vorteilhafte Weise ist die Komprimierungseinheit derart ausgeführt, dass Daten gelöscht werden, sofern das Entscheidungskriterium zumindest zu einem gewissen Grad erfüllt ist. Alternativ oder in Ergänzung ist es denkbar, dass in Abhängigkeit des Entscheidungskriterium oder dem Grad, mit dem das Entscheidungskriterium erfüllt wird, die übertragenen Daten komprimiert werden, bevorzugt, indem Teile von Datensätzen gelöscht werden.

Von Vorteil ist es, wenn das Entscheidungskriterium auf der Grundlage einer in einem Zeitintervall erfolgenden Veränderung eines Messwertes, einer Stärke einer Messwertänderung und/oder einer Anzahl von innerhalb eines Zeitraums erfasster Daten festgelegt wird. Das Entscheidungskriterium wird somit vorzugsweise in Abhängigkeit der zur Verfügung gestellten Daten gewählt, um entweder Daten, die im Vergleich zu bereits übertragenen Daten nur über wenig weiteren Informationsgehalt verfügen, nicht zu übertragen bzw. zu berücksichtigen oder aber besonders relevante Daten, also Daten mit einem besonders wichtigen Informationsgehalt, aus einer Gruppe von Daten auszuwählen und an die zentrale Datenverarbeitungseinheit oder einen Datenspeicher zu übertragen.

In einer weiteren speziellen Ausführungsform ist das Gasmessgerät als mobiles Gasmessgerät ausgebildet und verfügt über wenigstens ein Befestigungselement, das ein werkzeugfreies Befestigen und Lösen des Gasmessgerätes an einem Gürtel, einer Tasche, einem Kleidungsstück, einer Schutzausrüstung und/oder einem Helm ermöglicht. Im Weiteren kann ein derartiges Befestigungselement als Armband, Gürtel, Hüftgurt, Schultergurt oder Halsband ausgeführt sein, das eine Befestigung des Gasmessgeräts oberhalb der vom Messgeräteträger getragenen Kleidung ermöglicht.

Gemäß einer besonderen Ausgestaltung der Erfindung ist es denkbar, dass der Datenspeicher des Gasmessgeräts wenigstens zwei Speicherbereiche aufweist, in denen Daten, die jeweils spezifische Informationen enthalten, abgespeichert werden. Bevorzugt ist das Gasmessgerät, insbesondere ein mobiles oder tragbares Gasmessgerät, derart eingerichtet, dass die in den wenigstens zwei Speicherbereichen gespeicherten Daten getrennt voneinander übertragen werden. In diesem Zusammenhang ist es denkbar, dass sich die in den unterschiedlichen Speicherbereichen gespeicherten Daten in Bezug auf die Art der Datenerzeugung, die Herkunft, den Informationsgehalt und/oder die Datengröße unterscheiden. Besonders bevorzugt ist es denkbar, dass in einem ersten Speicherbereich des Datenspeichers eines Gasmessgeräts Messdaten abgelegt werden, während in wenigstens einem weiteren Speicherbereich während des Betriebs des Gasmessgeräts aufgetretene Fehler, generierte Alarme oder sonstige Informationen in Bezug auf die Bedienung oder den Service des Gerätes abgelegt sind. Durch das Vorsehen wenigstens zweier Speicherbereiche ist es möglich, unterschiedliche Daten in Bezug auf die Datenübertragung auf unterschiedliche Weise zu priorisieren und so etwa bestimmte Daten, wie etwa Informationen über Ereignisse, Alarme und/oder Messdaten, regelmäßig und in kurzen Zeitabständen auszulesen, während andere Daten, wie beispielsweise Kräfte, die während des Betriebs auf das Gasmessgerät eingewirkt haben, nur zu bestimmten Zeitpunkten, zum Beispiel während einer Wartung oder einer Kalibrierung, ausgelesen werden.

Im Übrigen sind auf vorteilhafte Weise wenigstens zwei Schnittstellen vorgesehen und das Gasmessgerät derart ausgebildet, dass in Abhängigkeit einer erforderlichen Sendeleistung und/oder Signalqualität eine der wenigstens zwei Schnittstellen für die Datenübertragung ausgewählt wird. Das Gasmessgerät verfügt in diesem Fall bevorzugt über wenigstens zwei Sendeeinheiten, sodass die für eine Datenübertragung erforderliche Paarung bestehend aus Sendeeinheit und Schnittstelle zwischen dem Gasmessgerät und der Datenverarbeitungseinheit hergestellt werden kann. Mit einer derartigen technischen Lösung wird sichergestellt, dass stets bedarfsgerecht die für eine zuverlässige und dennoch möglichst energiesparende Übertragung von Daten erforderliche Schnittstelle ausgewählt und genutzt wird. Das Vorsehen unterschiedlicher Schnittstellen ermöglicht darüber hinaus einen besonders flexiblen Betrieb und sicheren Betrieb eines Gasmessgerätes, insbesondere eines mobilen oder tragbare Gasmessgeräts, bei der Überwachung industrieller Produktionsstandorte mit einem erfindungsgemäß ausgeführten System zur Auswertung von Daten.

Neben einem System zur Auswertung von Daten betrifft die Erfindung auch ein mobiles Gasmessgerät zum Einsatz in einem System, das gemäß zumindest einer der zuvor beschriebenen Ausführungsformen ausgebildet ist.

In einer besonderen Ausgestaltung verfügt das mobile Gasmessgerät über wenigstens einen Datenspeicher. In dem Datenspeicher werden die generierten Daten rollierend abgespeichert, sodass, sobald kein Speicherplatz mehr zur Verfügung steht, die ältesten Daten auf dem Datenspeicher mit den gerade generierten Daten überschrieben werden.

Vorzugsweise weist der Datenspeicher wenigstens zwei Speicherbereiche auf, die getrennt oder gemeinsam mit der Schnittstelle zur Übertragung von Daten an die Datenverarbeitungseinheit verbindbar sind. Das Herstellen einer Verbindung zur Schnittstelle und damit mittelbar zur Datenverarbeitungseinheit wird durch eine Steuereinheit des mobilen Gasmessgeräts initiiert. Gemäß einer speziellen Weiterbildung der Erfindung wird die Verbindung zur Schnittstelle von der Steuereinheit auf der Grundlage eines Entscheidungskriteriums hergestellt. Mit Hilfe des vom Nutzer bedarfsgerecht festlegbaren Entscheidungskriterium ist es möglich, in Abhängigkeit bestimmter Ereignisse, beispielsweise der Größe von Messwerten, des Informationsgehalts der generierten Daten und/oder einem Abstand zwischen zwei nacheinander erfassten Messwerten eine Verbindung zur Schnittstelle herzustellen und/oder eine Auswahl des zu berücksichtigen Speicherbereichs des Datenspeichers zu treffen.

Ebenso betrifft die Erfindung eine Datenverarbeitungseinheit für ein erfindungsgemäß ausgeführtes System, das gemäß einer der zuvor beschriebenen Ausführungsformen gestaltet ist und das eingerichtet ist, um eine Konzentration wenigstens eines Gases zu erfassen und/oder zumindest eine Information über in einem zurückliegenden Zeitintervall aufgetretene Fehler, Alarme und/oder Kräfte, die auf das Gasmessgerät eingewirkt haben, zu erzeugen und diese Konzentration und/oder Information über eine Ausgabeeinheit auszugeben.

Im Folgenden wird die Erfindung ohne Beschränkung des allgemeinen Erfindungsgedankens anhand von Ausführungsbeispielen unter Bezugnahme auf die Figuren näher erläutert. Dabei zeigt:

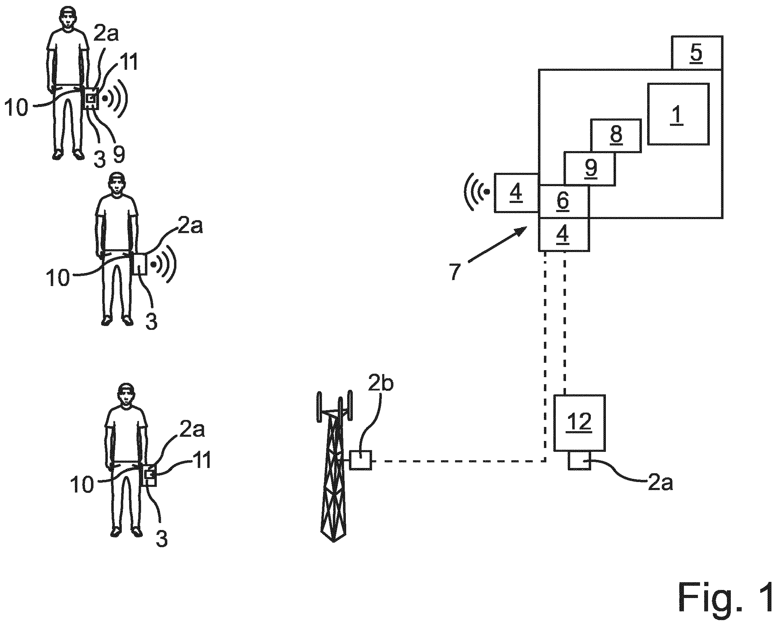

- Fig. 1:: Schematischen Schaltbild eines erfindungsgemäß ausgebildeten Systems zur Auswertung von Daten.

Fig. 1 zeigt in einem schematischen Schaltbild ein erfindungsgemäß ausgebildetes System zur Auswertung von Daten, wie es bei der Überwachung eines industriellen Produktionsstandortes und seiner unterschiedlichen Arbeitsbereiche auf das Vorhandensein unzulässiger Konzentrationen ausgewählter Gase oder Gasgemische eingesetzt wird. Die Daten werden von einer Vielzahl von Gasmessgeräten 2 bereitgestellt, die zu einem Teil stationär an unterschiedlichen Stellen des Produktionsstandortes, etwa einer Raffinerie, einem sonstigen Werk zur Herstellung von Chemikalien oder einem Bergwerk, montiert sind und zum anderen Teil von am Produktionsstandort Tätigen zu ihrer Absicherung mitgeführt werden. Bei den in Fig. 1 dargestellten Gasmessgeräten 2 handelt es sich somit sowohl um stationäre Gasmessgeräte 2b als auch um mobile oder tragbare Gasmessgeräte 2a, die an der Schutzkleidung oder am Körper des Geräteträgers mittels geeigneter Befestigungselemente 10 befestigbar sind. Während die tragbaren Gasmessgeräte 2a flexibel einsetzbar sind und vor allem dem Schutz des jeweiligen Gasmessgeräteträgers dienen, erfassen die stationären Gasmessgeräte 2b fortlaufend die Konzentration ausgewählter Dämpfe, Gase oder Gasgemische in einem Bereich des Produktionsstandorts, um sicherzustellen, dass in den jeweiligen Messbereichen keine gefährlichen Dampf- oder Gaskonzentrationen auftreten und die in diesem Bereich angeordneten Anlagen störungsfrei betrieben werden.

Die stationären Gasmessgeräte 2b übertragen in Abhängigkeit des Aufstellungsortes und der Befestigung des Gasmessgeräts 2b die erzeugten Messdaten sowie Daten, die Informationen in Bezug auf das Auftreten von Alarmen oder Gerätefehlern im Messzeitraum enthalten, zunächst entweder drahtgebunden oder drahtlos an eine Schnittstelle 4 und von dieser an eine zentrale Datenverarbeitungseinheit 1, die an einen zentralen Datenspeicher 5 als Zwischen- und/oder Ablagespeicher angebunden ist. In diesem Zusammenhang ist es denkbar, dass die über die Schnittstelle 4 übertragenen Daten unmittelbar nach erfolgter Datenübertragung verarbeitet, in bereits verarbeiteter oder noch unverarbeiteter Form an eine weitere Datenverarbeitungseinheit übertragen und/oder zunächst auf dem zentralen Datenspeicher 5 abgespeichert werden. Gemäß dem hier beschriebenen Ausführungsbeispiel ist die zentrale Datenverarbeitungseinheit 1 Teil einer Leitwarte, von der aus der Produktionsstandort, insbesondere die verschiedenen Produktionsanlagen, überwacht und bedarfsgerecht gesteuert wird.

Die in dem System zur Auswertung von Daten gemäß Fig. 1 verwendeten tragbaren Gasmessgeräte 2a lassen sich in zwei Klassen unterteilen. Einerseits handelt es sich um Gasmessgeräte 2a, die nicht in der Lage sind, eine drahtlose Datenübertragungsverbindung zu der zentralen Datenverarbeitungseinheit 1 herzustellen. Diese Gasmessgeräte 2a alarmieren den Geräteträger über eine Ausgabeeinheit 11, etwa ein Anzeigeelement oder ein Lautsprecher, zwar unmittelbar bei Detektion einer Überschreitung eines Grenzwerts für eine zulässige Gaskonzentration oder Unterschreitung einer erforderlichen Sauerstoffkonzentration, speichern die generierten Messdaten sowie eventuell erzeugte Daten, die Informationen über im Messzeitraum aufgetretenen Gerätefehler und/oder Alarme enthalten, aber zunächst nur auf einem internen Datenspeicher 3, einem sogenannten Datenlogger. Das Auslesen des internen Datenspeichers 3, also das Übertragen der auf diesem Datenspeicher gespeicherten Daten an die Schnittstelle 4, erfolgt üblicherweise mit Hilfe eines oftmals stationär betriebenen Auslesegeräts 12, an das das mobile Gasmessgerät 2a zeitweise angeschlossen wird, beispielsweise durch Einlegen in eine Geräteschale mit Kontakten. Das Auslesen der gespeicherten Daten erfolgt oftmals während des Ladens des Akkumulators, der Kalibrierung der geräteinternen Sensoren oder bei einer sonstigen Wartung, Reinigung oder Reparatur des Gasmessgeräts 2a.

Entweder ist in das Auslesegerät 12 eine geeignete Schnittstelle 4 integriert oder das Auslesegerät 12 ist drahtlos oder drahtgebunden mit der Schnittstelle 4 verbindbar, sodass die die aus dem Datenspeicher 3 des Gasmessgeräts 2a ausgelesenen Daten über die Schnittstelle 4 an die zentrale Datenverarbeitungseinheit 1 übertragen werden können.

Die Erzeugung der Daten erfolgte somit in zeitlichem Abstand vor der Datenübertragung, sodass die auf diese Weise der Datenverarbeitungseinheit 1 zur Verfügung gestellten Daten als historische Daten bezeichnet werden.

Bei der zweiten Klasse von gemäß der hier beschriebenen Ausführungsform verwendeten mobilen Gasmessgeräten 2a handelt es sich um Geräte, die in zunehmendem Maße zum Einsatz kommen und die zumindest in bestimmten Bereichen des Produktionsstandorts eine drahtlose Verbindung zu einer geeigneten Schnittstelle 4 herstellen, sodass die erzeugten Daten, insbesondere Messdaten, als aktuelle Daten in Echtzeit an die zentrale Datenverarbeitungseinheit 1 übertragen werden.

In diesem Zusammenhang ist es denkbar, dass derartige mobile Gasmessgeräte 2a während der gesamten Zeit, in der sich ein Geräteträger am Produktionsstandort bewegt, mit der Schnittstelle 4 zur Übertragung von Daten an die zentralen Datenverarbeitungseinheit 1 verbunden sind, oder aber, dass eine Datenübertragungsverbindung entweder in Abhängigkeit der Netzabdeckung nur in bestimmten Bereichen des Produktionsstandorts besteht oder aber nur in ausgewählten Bereichen gezielt hergestellt wird.

Die mobilen oder tragbaren Gasmessgeräte 2a sind wiederum derart ausgeführt, dass einerseits Messdaten und Daten, die Informationen über im Messzeitraum aufgetretene Gerätefehler oder Alarme enthalten, und eine Information über den Ort des Gasmessgeräts 2a über die Schnittstelle 4 an die zentrale Datenverarbeitungseinheit 1 übertragen werden und andererseits bei Detektion einer unzulässigen Grenzwertverletzung ein Alarmsignal über eine Ausgabeeinheit 11, etwa ein Anzeigeelement oder ein Lautsprecher, an den Geräteträger ausgegeben wird. Ein derartiges Alarmsignal wird in Form eines Alarmtons, einer Anzeige auf einem Display und/oder als Vibrationsalarm ausgegeben. Ergänzend ist es denkbar, dass im tragbaren Gasmessgerät 2a ein zusätzlicher Schwellwert hinterlegt ist, bei dessen Verletzung noch vor Erreichen des Grenzwerts ein Voralarm, der bevorzugt zumindest eine andere Eigenschaft als der Hauptalarm aufweist, ausgelöst wird.

Eine Information über den Aufenthaltsort des Gasmessgeräts 2a kann hierbei auf unterschiedliche Weise, etwa automatisiert mit Hilfe eines Gateways, über das vom Gasmessgerät 2a erzeugte Daten übertragen werden, durch manuelle Eingabe, Scannen eines RFID-Tags oder Auswertung eines GPS-Signals erzeugt und den übertragenen Daten hinzugefügt werden. Ebenso ist es denkbar, dass eine weitere Systemkomponente, die eine Information über den Aufenthaltsort des Gasmessgeräts 2a besitzt, die übertragenen Daten um eine Ortsinformation ergänzt oder sogar, dass eine Ortsinformation aufgrund bekannter Bewegungsinformationen ermittelt und den übertragenen Daten hinzugefügt wird.

In dem in Fig. 1 gezeigten System kommen somit sowohl stationäre als auch mobile oder tragbare Gasmessgeräte 2b, 2a zum Einsatz, wobei die mobilen Gasmessgeräte 2a zum Teil historische Daten und zum Teil aktuelle Daten über die Schnittstelle 4 an die Datenverarbeitungseinheit 1 übertragen. Selbstverständlich kann sich die Zusammensetzung der im Betrieb befindlichen Gasmessgeräte stets ändern. Aus diesem Grund muss es unabhängig von der Art der bereitstellten Daten, also unabhängig davon, ob es sich um historische oder aktuelle Daten handelt, zu jeder Zeit möglich sein, die Daten eindeutig zu unterscheiden und die Zeitpunkte ihrer Generierung in den korrekten zeitlichen Zusammenhang zu setzen. Es ist somit wesentlich, dass in dem hier beschriebenen System zur Auswertung von bereitgestellten Daten sichergestellt ist, dass gleichgültig ob es sich um historische oder aktuelle Daten handelt, stets der Zeitpunkt ihrer Entstehung und die korrekte zeitliche Abfolge der Entstehungszeitpunkte bei der Datenverarbeitung berücksichtigt wird. Die von den verschiedenen Gasmessgeräten 2 bereitgestellten Daten müssen daher so aufbereitet werden, dass ihr Informationsgehalt nicht verlorengeht und die korrekte zeitliche Reihenfolge ihrer Entstehung berücksichtigt werden kann.

Gemäß der in Fig. 1 gezeigten Ausführungsform wird dieses Problem dadurch gelöst, dass zumindest eine Schnittstelle 4 vorgesehen ist, die eine Meldung an das mit der Schnittstelle verbundene Gasmessgerät 2 mit der Information sendet dass das jeweilige Gasmessgerät 2 nun mit der Schnittstelle 4 verbunden ist und ab diesem Zeitpunkt eine Übertragung von Daten an die zentrale Datenverarbeitungseinheit erfolgt. Die Information über die Herstellung der Verbindung wird darüber hinaus als Kennzeichnung im Datenspeicher 3 des Gasmessgerätes abgelegt. Sofern in diesem Datenspeicher 3 bereits historische Daten abgelegt sind, erfolgt deren Kennzeichnung derart, dass eindeutig ist, ab wann historische Daten aus dem Datenspeicher 3 des Gasmessgeräts 2 ausgelesen werden. Sobald die Verbindung zwischen der Schnittstelle 4 und dem Gasmessgerät 2 wieder getrennt wird, gleichgültig ob dies gewollt oder ungewollt passiert, erzeugt das Gasmessgerät 2 einen weiteren Eintrag im Datenspeicher 3 des Gasmessgeräts 2, der eine Information über den Verbindungsabbruch insbesondere über dessen Zeitpunkt enthält. Vorzugsweise wird jedem Eintrag eine eindeutige ID hinzugefügt, sodass stets unterschieden werden kann, ob die Daten bereits vorliegen, wobei es sich bei der ID um einen Zeitstempel oder einen Counter handeln kann. Einerseits ist auf dies Weise in den Daten die Information über den Zeitpunkt der Trennung oder Herstellung einer Verbindung zur Datenübertragung enthalten und andererseits werden Doppeleinträge vermieden.

Wird im Anschluss daran erneut eine Verbindung zwischen der Schnittstelle 4 und dem Gasmessgerät 2, insbesondere einem mobilen oder tragbaren Gasmessgerät 2a, hergestellt, ist auf diese Weise sichergestellt, dass die Daten die zwischen den beiden Kennzeichnungen auf dem Datenspeicher 3 des Gasmessgeräts 2 abgelegt wurden, nicht noch einmal an die Datenverarbeitungseinheit 1 übertragen werden bzw. von dieser für die weitere Verarbeitung nicht berücksichtigt werden.

Im Weiteren ist das in Fig. 1 schematisch gezeigte System zur Auswertung von Daten derart ausgeführt, dass die von den Gasmessgeräten 2 verwendeten unterschiedlichen Zeiten berücksichtigt werden, wobei die unterschiedlichen Zeiten auf eine einheitliche Systemzeit synchronisiert werden. Hierfür sind gemäß der Fig. 1 gezeigten Ausführungsforum die Schnittstelle 4 und eine Datenkennzeichnungseinheit 6 Teil eines Gateways 7, das zunächst die jeweils von einem Gasmessgerät 2 übertragenen mit einer Information über die Systemzeit anreichert, also einen sogenannten Zeitstempel hinzufügt. Um sicherzustellen, dass stets eine einheitliche Systemzeit verwendet wird, wird das Gateway 7 mit einer vorhandenen Systemzeit synchronisiert. Ebenso fügt ein Gasmessgerät 2 bei einer Datenübertragung den übertragenen Daten eine Kennzeichnung, die eine Information über die Gasmessgerätezeit zu diesem Zeitpunkt enthält, bei. Werden nunmehr historische Daten aus Gasmessgeräten 2, insbesondere aus mobilen Gasmessgeräten 2a, ausgelesen und über das Gateway 7 an die zentrale Datenverarbeitungseinheit 1 übertragen, werden mittels einer Kompensationseinheit 8, die in die zentrale Datenverarbeitungseinheit 1 integriert sein kann, die Erfassungszeitpunkte von der Gasmessgerätezeit in die Systemzeit umgerechnet. Die Verarbeitung, Auswertung und/oder die Analyse der übertragenen Daten erfolgt somit immer auf Basis der Systemzeit, und das unabhängig davon, ob es sich um aktuelle oder historische Daten handelt und ob die jeweiligen Gasmessgerätezeiten mit der Systemzeit übereinstimmen.

Wesentlich für das beschriebene System ist, dass eine große Menge von Daten, und zwar sowohl historische als auch aktuelle einer zentralen Datenverarbeitung zur Verfügung gestellt und hier verarbeitet bzw. ausgewertet werden. Aufgrund der großen Datenmenge, die für Auswertungen und Analysen zur Verfügung steht, können mit großer Genauigkeit und hoher Ortsauflösung für den überwachten Produktionsstandort Aussagen über bestehende Risiken und/oder über stattgefundene Ereignisse, beispielsweise Leckagen oder Unfälle, gemacht werden. Hierbei ist allerdings zu berücksichtigen, dass in dem beschriebenen System teilweise erhebliche Datenmengen übertragen, abgespeichert und/oder verarbeitet werden müssen. Aus diesem Grund ist es sinnvoll, die Menge der übertragenen Daten dahingehend zu reduzieren, dass nur die für die spätere Verarbeitung und Auswertung wirklich relevanten Daten übertragen und später genutzt werden. In diesem Zusammenhang sind die Gasmessgeräte 2, insbesondere die mobilen Gasmessgeräte 2a, derart ausgeführt, dass nur bestimmte Messdaten im Datenspeicher 3 eines Gasmessgeräts 2 abgelegt und/oder an die Schnittstelle 4 übertragen werden.

Bevorzugt wird ein Messwert nur abgespeichert und/oder übertragen, wenn ein in einem Steuergerät des Gasmessgeräts 2 hinterlegtes Entscheidungskriterium zumindest zu einem festlegbaren Grad erfüllt oder nicht erfüllt ist. Durch das Entscheidungskriterium wird hierbei auf vorteilhafte Weise unter anderem berücksichtigt, ob eine Datenverbindung zu einer zentralen Datenverarbeitungseinheit und/oder einem zentralen Datenspeicher besteht. Eine Speicherung und/oder Übertragung von Daten erfolgt beispielsweise nur dann, wenn ein Messwert ober- oder unterhalb eines Grenzwerts liegt, zwischen zwei Messungen eine definierte Zeitspanne liegt, ein mobiles Gasmessgerät 2a über eine bestimmte Entfernung bewegt worden ist und/oder zwei nacheinander aufgenommene Messwerte einen festgelegten Abstand zueinander aufweisen. Auf diese Weise ist es durch Einsatz einer Komprimierungseinheit 9 möglich, unter Berücksichtigung wenigstens eines Entscheidungskriteriums eine Speicherung und/oder Übertragung von Daten zu begrenzen und/oder bereits abgespeicherte oder übertragene Daten zumindest teilweise zu löschen, um die Datenmenge auf diese Weise zumindest teilweise zu komprimieren. Die Festlegung des wenigstens einen Entscheidungskriteriums, das einer Reduzierung der Daten, die gespeichert, übertragen und/oder verarbeitet werden, zugrunde gelegt wird, kann angepasst an die jeweiligen Betriebsbedingungen, insbesondere das Gefahrenpotenzial, das für einen Gasmessgeräteträger besteht, festgelegt werden.

Im Übrigen ist es hierbei ebenfalls möglich, die Eigenschaften der Sensoren sowie der Messverfahren, die in einem Gasmessgerät zum Einsatz kommen, zu berücksichtigen. Beispielsweise ist nicht immer die Abweichung eines Messwerts in Bezug auf einen Nullpunkt von Bedeutung, sondern, wie etwa beim Sauerstoffgehalt der Luft von 20,9 Vol%, die Abweichung von einem spezifischen Wert.

Im Weiteren sind die Datenspeicher 3 der Gasmessgeräte 2 derart ausgeführt, dass wenigstens zwei unterschiedliche Speicherbereiche vorgesehen sind, in denen jeweils Daten mit unterschiedlichem Informationsgehalt abgelegt werden. Auch in diesem Fall ist es wiederum denkbar, die Speicherbereiche bedarfsgerecht festzulegen, um beispielsweise Messwerte, Informationen über Alarme, Fehlermeldungen oder andere Ereignisse in getrennten, jeweils hierfür vorgesehene Speicherbereichen abzulegen. Durch das Vorsehen derart getrennter Speicherbereiche und einer geeignet ausgeführten Ansteuerung der Datenspeicher 3 ist es sogar möglich, die Datenspeicher 3 der Gasmessgeräte 2 adaptiv auszulesen und somit die Übertragung von Daten an die Schnittstelle 4 zu bestimmten Zeitpunkten durchzuführen und/oder auf bestimmte Zeiträume oder Ereignisse zu beschränken. So ist es etwa denkbar, größere Speicherbereiche oder Speicherbereiche, in denen weniger sicherheitsrelevante Daten gespeichert sind, in vergleichsweise großen Zeitabständen oder nur bei bestimmten Ereignissen, etwa bei einer Wartung oder Kalibrierung eines Gasmessgeräts 2, auszulesen, während kleinere Speicherbereiche oder Speicherbereiche mit besonders sicherheitsrelevanten Informationen, beispielsweise Messwerten in Grenzwertnähe oder Alarmereignisse, häufiger ausgelesen werden.

Der wesentliche Vorteil des erfindungsgemäßen Systems zur Auswertung von Daten besteht darin, dass mit geeignet ausgebildeten Gasmessgeräten 2 und sowohl zumindest einer entsprechend eingerichteten Schnittstelle 4 als auch einer Datenverarbeitungseinheit 1 historische und auch aktuelle Daten für eine Datenauswertung und -analyse zur Verfügung stehen. Aufgrund des beschriebenen Systems ist es möglich, sicherheitsrelevante Informationen über die an einem Produktionsstandort herrschenden Gaskonzentrationen und Umgebungsparameter mit großer Genauigkeit, hoher Ortauflösung und über einen vergleichsweise großen Bereich zur Verfügung zu stellen. Die herrschende Gesamtsituation kann so besser bewertet, insbesondere eine zuverlässige Risikobeurteilung durchgeführt werden. Berücksichtigung findet hierbei die gemessene Konzentration ausgewählter Gase, die Anzahl der in einem Messzeitraum aufgetretenen Alarme sowie die Häufung bestimmter Fehler.

Im Weiteren wird wegen der großen zur Verfügung stehenden Datenmenge eine eingehende Datenanalyse ermöglicht, die mit hoher Wahrscheinlichkeit Rückschlüsse auf Eigenschaften und/oder die Eigenschaftsveränderungen eines Gasmessgeräts 2, die durch die erfolgte Begasung der verwendeten Sensoren, während der Messungen herrschende Umweltbedingungen und das Alter der Sensoren bedingt sein können, zulässt.

Durch die geeignete Kennzeichnung der von den verschiedenen Gasmessgeräten 2 generierten und zur Verfügung gestellten historischen und aktuellen Daten kann eine vergleichsweise große Menge von Daten präzise analysiert werden. Hierbei wird mithilfe des erfindungsgemäßen Systems sichergestellt, dass eine doppelte Auswertung von Daten unterbleibt, und zwar einerseits dadurch, dass in den Datenspeichern 3 der Gasmessgeräte 2 Kennzeichnungen in Bezug auf den Beginn und das Ende einer Datenübertragung bzw. die Herstellung und den Abbruch einer Datenverbindung, vorgenommen werden und darüber hinaus den jeweils übertragenen Daten eindeutige Zeitstempel hinzugefügt werden, sodass sämtliche in der Datenverarbeitungseinheit 1 verwendeten Daten auf die Systemzeit synchronisiert sind. Ebenso kann den Daten eine eindeutige ID hinzugefügt werden, um stets eine eindeutige Identifizierung der Daten zu ermöglichen.

### Bezugszeichenliste

- 1: Datenverarbeitungseinheit
- 2: Gasmessgerät
2a mobiles Gasmessgerät
2b stationäres Gasmessgerät

- 3: Datenspeicher im Gasmessgerät
- 4: Schnittstelle
- 5: zentraler Datenspeicher
- 6: Datenkennzeichnungseinheit
- 7: Gateway
- 8: Kompensationseinheit
- 9: Komprimierungseinheit
- 10: Befestigungselement
- 11: Ausgabeeinheit
- 12: Auslesegerät

## Patentansprüche

1. System zur Auswertung von Daten, mit wenigstens einem Gasmessgerät (2), das eingerichtet ist, um aktuelle Daten und/oder historische Daten, die in einem Datenspeicher (3) des Gasmessgeräts (2) abgelegt sind und eine Information über eine Gasmessgerätzeit zum Erfassungszeitpunkt der Daten enthalten, bereitzustellen und drahtgebunden oder drahtlos an eine Schnittstelle (4) zu übertragen, die ausgeführt ist, um die aktuellen und/oder die historischen Daten zu empfangen und einer Datenverarbeitungseinheit (1) und/oder einem zentralen Datenspeicher (5) zuzuführen, und mit einer Datenkennzeichnungseinheit (6), die ausgeführt ist, um den von dem Gasmessgerät (2) bereitgestellten Daten wenigstens zeitweise eine spezifische Kennzeichnung hinzufügen,
**dadurch gekennzeichnet, dass** die Datenkennzeichnungseinheit (6) eingerichtet ist, um den von dem Gasmessgerät (2) bereitgestellten Daten eine Information über eine Systemzeit während der Datenübertragung und/oder die Gasmessgerätezeit während der Datenübertragung hinzuzufügen und dass die Datenverarbeitungseinheit (1) derart ausgeführt ist, dass eine Verarbeitung der vom Gasmessgerät (2) und/oder dem zentralen Datenspeicher (5) bereitgestellten Daten unter Berücksichtigung der Systemzeit und der Gasmessgerätezeit erfolgt.

2. System nach Anspruch 1,
**dadurch gekennzeichnet, dass** die Schnittstelle (4) und die Datenkennzeichnungseinheit (6) in ein Gateway (7) integriert sind.

3. System nach Anspruch 1 oder 2,
**dadurch gekennzeichnet, dass** die Schnittstelle (4) eingerichtet ist, um beim Herstellen einer Datenverbindung zwischen dem Gasmessgerät (2) und der Schnittstelle (4) eine Information über die erfolgte Verbindungsherstellung an das Gasmessgerät (2) zu übertragen.

4. System nach Anspruch 3,
**dadurch gekennzeichnet, dass** das Gasmessgerät (2) ausgebildet ist, um die Information über die erfolgte Verbindungsherstellung im Datenspeicher (3) des Gasmessgeräts (2) zu speichern und um bei Trennung der Datenverbindung zwischen Gasmessgerät (2) und Schnittstelle (4) eine Information über die erfolgte Trennung zu erzeugen und in dem Datenspeicher (3) des Gasmessgeräts (2) zu speichern.

5. System nach einem der vorangehenden Ansprüche,
**dadurch gekennzeichnet, dass** eine Kompensationseinheit (8) vorgesehen ist, die eingerichtet ist, um den Erfassungszeitpunkt gemäß Gasmessgerätezeit der historischen Daten unter Berücksichtigung der System- und der Gasmessgerätezeit bei der Datenübertragung in einen Erfassungszeitpunkt gemäß Systemzeit umzurechnen und den jeweiligen Daten zuzuordnen.

6. System nach einem der vorangehenden Ansprüche,
**dadurch gekennzeichnet, dass** eine Komprimierungseinheit (9) vorgesehen ist, die eine Größe und/oder eine Menge der vom Gasmessgerät (2) zur Verfügung gestellten Daten in Abhängigkeit wenigstens eines Entscheidungskriteriums verringert.

7. System nach Anspruch 6,
**dadurch gekennzeichnet, dass** die Komprimierungseinheit eingerichtet ist, um Daten zu löschen, sofern das Entscheidungskriterium nicht erfüllt ist.

8. System nach Anspruch 6 oder 7,
**dadurch gekennzeichnet, dass** das Entscheidungskriterium eine in einem Zeitintervall erfolgende Veränderung eines Messwertes, eine Stärke einer Messwertveränderung und/oder eine Anzahl innerhalb eines Zeitraums erfasster Daten berücksichtigt.

9. System nach einem der vorangehenden Ansprüche,
**dadurch gekennzeichnet, dass** der Datenspeicher (3) des Gasmessgeräts (2) wenigstens zwei Speicherbereiche aufweist, in denen jeweils Daten in Bezug auf spezifische Ereignisse abgespeichert sind und das Gasmessgerät (2) derart eingerichtet ist, dass die in den wenigstens zwei Speicherbereichen gespeicherten Daten getrennt voneinander übertragbar sind.

10. System nach einem der vorangehenden Ansprüche,
**dadurch gekennzeichnet, dass** wenigstens zwei Schnittstellen (4) vorgesehen sind, und das Gasmessgerät (2) ausgebildet ist, um in Abhängigkeit einer für die Datenübertragung erforderlichen Sendeleistung und/oder Signalqualität eine der wenigstens zwei Schnittstellen (4) für die Datenübertragung auszuwählen.

11. Mobiles Gasmessgerät (2a) für ein System nach wenigstens einem der vorangehenden Ansprüche,
**dadurch gekennzeichnet, dass** wenigstens ein Datenspeicher (3) zur Bereitstellung der Daten vorgesehen ist, auf dem Daten rollierend speicherbar sind.

12. Mobiles Gasmessgerät nach Anspruch 11,
**dadurch gekennzeichnet, dass** der Datenspeicher (3) wenigstens zwei Speicherbereiche aufweist, die getrennt oder gemeinsam mit der Schnittstelle (4) zur Übertragung von Daten an die Datenverarbeitungseinheit verbindbar sind.

13. Mobiles Gasmessgerät nach Anspruch 11 oder 12,
**dadurch gekennzeichnet, dass** eine Steuereinheit vorgesehen ist, durch die auf der Grundlage eines Entscheidungskriteriums eine Verbindung zur Schnittstelle (4) zum Übertragen der im Datenspeicher (3) gespeicherten Daten an die zentrale Datenverarbeitungseinheit (1) herstellbar ist.

14. Datenverarbeitungseinheit (1) für ein System nach wenigstens einem der Ansprüche 1 bis 10, das eingerichtet ist, um aus den erfassten Daten eine Konzentration wenigstens eines Gases, eine Anzahl von in einem Zeitintervall erfolgten Alarmen oder Fehlmessungen und/oder Kräfte, die in einem zurückliegenden Zeitraum auf das Gasmessgerät (2) eingewirkt haben, zu ermitteln und über eine Ausgabeeinheit (11) auszugeben.
